# FASCICULE DE BREVET EUROPEEN

(11) **EP 1 694 255 B1**
(45) Date de publication et mention de la délivrance du brevet: **11.04.2007**
(21) Numéro de dépôt: 04805644.4
(22) Date de dépôt: 06.12.2004
(51) Int. Cl.: A61F 2/28

(54) **PROCEDE DE PURIFICATION DU CORAIL, AINSI QUE LE CORAIL OBTENU PAR CE PROCEDE**
KORALLENREINIGUNGSVERFAHREN UND SO ERHALTENE KORALLE
CORAL PURIFICATION METHOD AND CORAL THUS OBTAINED

(30) Priorité: 05.12.2003 FR 0314299
(43) Date de publication de la demande: 30.08.2006
(73) Titulaire: BIO HOLDINGS INTERNATIONAL LIMITED, Tortola (VG)
(72) Inventeur: LE PETITCORPS, Yann, F-33850 Leognan (FR); FRICAIN, Jean-Christophe, F-33000 Bordeaux (FR); SOUILLAC, Vincent, F-33000 Bordeaux (FR); LARGETEAU, Alain, F-33610 Cestas (FR); SCHMITTHAEUSLER, Roland, F-78180 Montigny Le Bretonneux (FR)
(74) Mandataire: Hubert, Philippe
(86) Numéro de dépôt international: PCT/FR2004/003126
(87) Numéro de publication internationale: WO 2005/055885

(56) Documents cités:
- EP-A- 0 603 920
- WO-A-99/02200
- US-A- 5 723 012
- US-B1- 6 414 050
- US-B1- 6 579 532
- DATABASE WPI Section Ch, Week 199921 Derwent Publications Ltd., London, GB; Class D22, AN 1999-244730 XP002291521 & CN 1 203 189 A (UNIV ZHONGSHAN OPHTHALMOLOGY CENT ZHONGS) 30 décembre 1998 (1998-12-30)
- DATABASE WPI Section Ch, Week 200358 Derwent Publications Ltd., London, GB; Class D22, AN 2003-608624 XP002291522 & CN 1 416 910 A (YIHUAJIAN SCI & TRADE CO LTD BEIJING) 14 mai 2003 (2003-05-14)
- DATABASE WPI Section Ch, Week 200102 Derwent Publications Ltd., London, GB; Class B04, AN 2001-007912 XP002291523 & CN 1 144 102 A (GUIZHOU PROV BIOLOGICAL RESOURCE SUPERCR) 5 mars 1997 (1997-03-05)

## Description

La présente invention concerne un procédé de purification du corail, ainsi que le corail obtenu par ce procédé.

L'invention trouve notamment application dans le domaine médical.

Le corail, qui présente une structure poreuse, est composé essentiellement (environ 97 %) de matière minérale, majoritairement de carbonate de calcium, le reste (environ 3 %) étant constitué d'oxydes de magnésium et de fer ainsi que de substances organiques responsables de sa couleur. Sa dureté varie de 2,5 à 3,5 sur l'échelle de Mohs.

Le carbonate de calcium constitutif du corail présente une structure cristallographique métastable de type aragonite susceptible de se transformer facilement en calcite, notamment sous l'effet de la température autour de 260°C.

La matière organique du corail contient une matière protéique mais est sensiblement exempte de matière lipidique.

Il existe à ce jour plusieurs substituts osseux à base de corail autorisés par l'Agence Française de Sécurité Sanitaire des Produits de Santé (AFSSAPS). Un de ces produits, commercialisé par la Société INOTEB sous la dénomination BIOCORAL®, est obtenu par traitement du corail à la surface de ses pores, ce qui permet d'éliminer une partie des substances organiques mentionnées ci-dessus.

Une analyse morphologique plus poussée du corail (microscopie électronique à balayage et thermique couplée à la spectroscopie de masse) montre toutefois que lesdites substances organiques sont présentes non seulement dans les pores du corail, mais également aux joints de grains des particules de carbonate de calcium. Le traitement mis en place pour le produit BIOCORAL®, ne permet pas d'extraire les substances organiques localisées aux joints de grains, qui représentent environ 90 % de la totalité des substances organiques.

Un problème que vise à résoudre la présente invention est donc de disposer de corail sensiblement exempt de matières organiques et en particulier de protéines qui puisse être utilisé pour fabriquer des substituts osseux de qualité comparable à celle des implants synthétiques existants.

Par « corail sensiblement exempt de matières organiques », on entend dans la présente description du corail contenant au moins deux fois moins de substances organiques que le corail non traité.

Un autre problème que vise à résoudre l'invention est de disposer d'un procédé qui permette d'éliminer les substances organiques présentes dans le corail sans modifier la structure cristalline de ce dernier.

Il a maintenant été découvert, et c'est le fondement de l'invention, que le traitement du corail par un fluide en conditions supercritiques, dans un domaine de température et de pression optimisé, permet d'apporter une solution aux problèmes énoncés ci-dessus, qui n'ont pas été résolus par les procédés connus de l'état de la technique.

Ainsi, selon un premier aspect, l'invention a donc pour objet un procédé d'extraction des substances organiques présentes dans le corail, qui consiste à traiter le corail par un fluide ou un mélange de fluides à l'état supercritique sans modifier la structure cristalline dudit corail, à une température inférieure à 270°C, de préférence inférieure ou égale 260°C, et de préférence encore inférieure ou égale à 250°C, et à une pression très supérieure à la pression critique dudit fluide ou mélange de fluides, par exemple de l'ordre d'au moins 3 fois, et de préférence d'au moins 5 fois ladite pression critique.

L'utilisation d'un fluide à l'état supercritique pour extraire des substances organiques a été décrite dans le document EP 0 603 920. Cependant, ce document concerne uniquement le traitement d'un tissu osseux et utilise le dioxyde de carbone pour extraire les lipides, c'est-à-dire des substances que l'on ne trouve pas dans le corail. Pour extraire les protéines, on fait appel selon ce document antérieur à un traitement complémentaire de nature chimique ou enzymatique à l'aide de composés additionnels tels que détergents ou oxydants, qui seraient susceptibles d'altérer la structure du corail. En outre, selon ce document, le traitement est réalisé à une pression voisine de la pression critique du dioxyde de carbone.

Selon le procédé de l'invention, on utilise donc un fluide dans des conditions dites "supercritiques", c'est-à-dire que le fluide possède les propriétés invasives d'un gaz tout en se comportant comme un liquide. De ce fait, le fluide est capable de diffuser dans la structure poreuse du corail sans aucun problème de mouillabilité, ce qui permet d'optimiser l'extraction des substances organiques et en particulier les protéines (que l'on désignera également par la suite par "matrice organique") présentes dans le corail.

Le fluide ou le mélange de fluides susceptible d'être utilisé dans le procédé de l'invention doit avoir une température critique inférieure à 270°C, de préférence inférieure ou égale à 260°C, et de préférence encore inférieure ou égale à 250°C, et permettre la solubilisation des matières organiques.

A titre d'exemple, on peut citer l'éthanol, l'acétone, ou un mélange d'un de ces deux fluides avec du dioxyde de carbone possédant une température critique inférieure à 270°C. Avantageusement, on utilise de l'éthanol du fait de la disponibilité de ce produit et de son caractère peu onéreux et non-toxique. La température critique de l'éthanol est de 240°C et sa pression critique est de 6,12 MPa.

Dans le cas où le fluide est de l'éthanol ou de l'acétone, la température de traitement du corail est avantageusement comprise entre 240 et 260°C.

Dans le cas où l'on utilise un mélange de fluides tel qu'un mélange éthanol-dioxyde de carbone, la température de traitement du corail est avantageusement comprise entre 80 et 100°C.

Le procédé de l'invention est mis en oeuvre à une pression suffisante, très supérieure à la pression critique du fluide ou du mélange de fluides utilisé pour éviter la transformation de la structure cristalline du corail. De ce point de vue, la pression critique des fluides mentionnés ci-dessus, est facilement accessible.

Dans le cas de l'éthanol ou de l'acétone, la pression de traitement du corail est avantageusement comprise entre 300 et 450 MPa, de préférence entre 350 et 450 MPa.

Dans le cas d'un mélange de fluides tel qu'un mélange éthanol-dioxyde de carbone, la pression de traitement du corail est avantageusement comprise entre 30 et 50 MPa, de préférence de l'ordre de 40 MPa.

La durée du traitement n'est pas critique en soi et varie généralement entre quelques minutes et quelques heures, en fonction du fluide utilisé. Dans le cas de l'éthanol ou de l'acétone, cette durée est avantageusement comprise entre 15 et 240 min, de préférence d'une heure environ.

Etant donné la composition chimique minérale du corail, le fluide ou le mélange de fluides selon l'invention est avantageusement utilisé sans aucun composé additionnel tel qu'un détergent ou un oxydant susceptible d'altérer la structure du corail.

Selon un second aspect, l'invention concerne le corail sensiblement exempt de substances organiques, tel qu'obtenu par le procédé décrit ci-dessus.

Selon un troisième aspect, l'invention concerne les substituts osseux réalisés à partir de corail sensiblement exempt de substances organiques. Ces substituts osseux peuvent être obtenus par, des méthodes bien connues de l'homme du métier.

L'invention va maintenant être illustrée à l'aide des exemples ci-après.

### Exemple1 : Traitement supercritique du corail

Le corail brut est traité dans un dispositif de génération de haute pression d'eau dans une enceinte ayant un volume fixé. Celle-ci, réalisée dans un alliage à hautes caractéristiques mécaniques, a une profondeur de 229 mm, un diamètre interne de 55 mm. Elle est fermée par un obturateur en forme de champignon supportant le joint métallique qui assure l'étanchéité par sa déformation. L'étanchéité au démarrage (sans pression dans l'autoclave) est réalisée par la remontée de l'obturateur, à l'aide de 8 vis dans la partie supérieure. La pression appliquée dans l'enceinte par la pompe ou la température va comprimer le joint qui va de lui-même rendre étanche le système.

La température est mesurée à l'intérieur de l'enceinte par un thermocouple monté dans la partie haute du réacteur. Il est en contact direct avec le fluide sous pression.

Le corail est placé dans des flacons souples de 20 ml en Téflon PFA (Réf. M77400, Fisher Bioblock Scientific) résistant à des températures avoisinant les 300°C et supportant la déformation due à la pression. La pression s'exerçant sur les parois internes et externes du flacon est la même. En effet, le flacon ne contenant pas ou très peu d'air, le liquide présent à l'intérieur du flacon et le liquide transmetteur (eau) sont à la même pression, la paroi est donc comme « invisible » et ne subit qu'une légère déformation due à la compressibilité de l'eau. Le traitement s'effectue en présence d'éthanol comme fluide "supercritique", à une température de 240 à 260°C, de préférence de l'ordre de 250°C sous une pression de 300 à 400 MPa, de préférence de l'ordre de 350 MPa, pendant 15 à 240 min, de préférence d'environ 1 h.

### Exemple 2 : Détermination de la quantité de substances organiques résiduelles dans le corail traité

L'échantillon obtenu à l'exemple 1 est broyé au mortier et pesé (masse MB - matière brute - d'environ 3 g). On prélève 3 g de la poudre obtenue que l'on dissous dans 135 ml d'HCl 0,1 N, sous agitation pendant 1 h. La solution ainsi obtenue est dialysée sur une membrane de poids moléculaire 12 000 à 16 000 (référence MCWO), puis congelée à -80°C et lyophilisée. Le lyophilisat est à son tour pesé (masse MO - matière organique - d'environ 3 g).

A titre de comparaison, la même expérience est réalisée sur un échantillon de corail brut (aragonite) et sur un échantillon de produit BIOCORAL®.

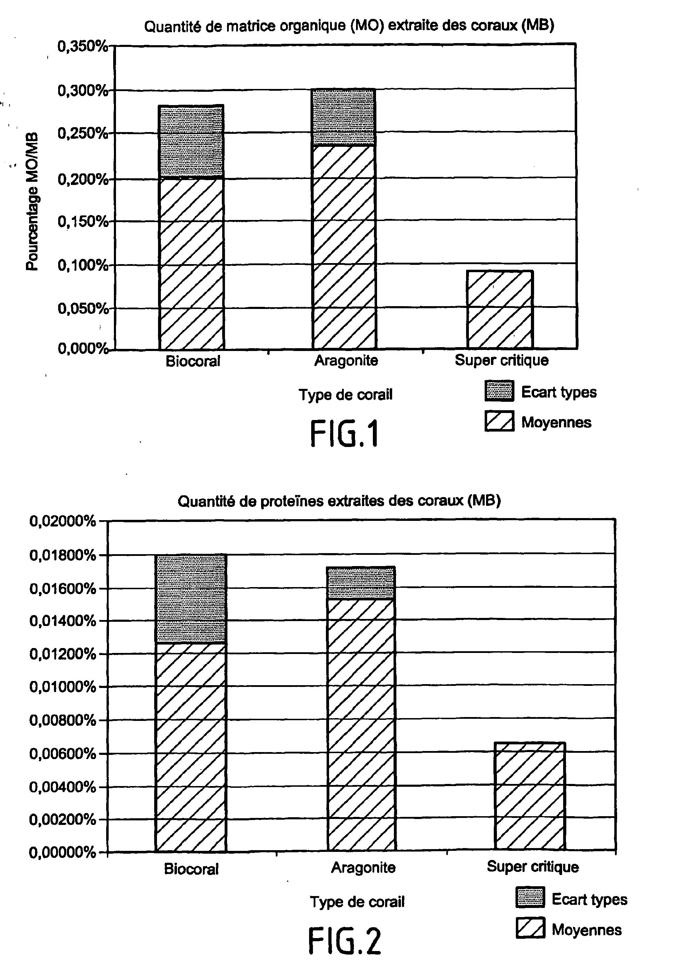

La figure 1 représente le rapport MO/MB pour chacun des trois échantillons. On peut constater que le traitement "supercritique" selon l'invention permet d'obtenir du corail contenant environ deux fois moins de substances organiques que le produit BIOCORAL®.

### Exemple 3 : Détermination de la quantité de protéines résiduelles dans le corail traité

### 1/ Préparation de la solution de travail et des échantillons :

- On mélange 50 volumes de la solution A avec 1 volume de la solution B. La solution de travail est légèrement trouble au début, puis rapidement elle s'éclaircie avec agitation douce au vortex. Sa couleur est vert opalescent.
- On broye les échantillons au mortier et on pèse la poudre obtenue.
- On dissout 1 g de poudre dans 45 ml d'HCL à 0,6 N pendant 1 heure sous agitation.

### 2/ Procédure pour la détermination :

- On dose la fraction soluble dans l'eau : 1,2 mg de matière organique sont solubilisés dans 75 µL d'eau.
- On solubilise le culot restant après centrifugation dans 75 µL de NaOH à 0,3 N.
- On prélève 25 µl de chaque standard (0 µg/ml, 5 µg/ml, 25 µg/ml, 50 µg/ml et 250 µg/ml) de chaque échantillon (préparés de la même manière que les échantillons à doser) et on les distribue dans les puits d'une plaque de 96 puits.
- On couvre la plaque et on incube pendant 30 min. à 37°C.
- On lit la densité optique à 562 nm une fois que la plaque est refroidie à température ambiante.

A titre de comparaison, la même expérience est réalisée sur un échantillon de corail brut (aragonite) et sur un échantillon de produit BIOCORAL®.

La figure 2 représente le rapport protéines/MB pour chacun des trois échantillons. On peut constater que le traitement "supercritique" selon l'invention permet d'obtenir du corail duquel environ deux fois plus de protéines ont été extraites, comparé au produit BIOCORAL®.

### Exemple 4 : Mise en évidence de la prolifération cellulaire de cellules osseuses sur le corail traité

On découpe des cylindres (∅=12 mm) en disque d'un mm d'épaisseur à l'aide d'une scie diamantée, puis on les stérilise par chaleur sèche à 100°C pendant 1h30 (3 cycles). Les disques sont déposés sur un lit d'aragose 0,1 % dans des boites de culture (NUNC ; 24 puits, 2 cm²). La suspension cellulaire est déposée sur chaque disque à raison de 20 000 cellules MG63/cm² (1,13 cm² x 2 car porosité - 22 600 cellules soit environ 50 000 cellules par échantillon), dans un volume de 100 µL de milieu de culture complet (IMDM : Iscowe's Modified Dubelcco's Medium, 10 % SVF - sérum de veau foetal). Après 30 minutes pour laisser les cellules adhérer, on complète avec 1 ml de milieu. Le milieu est changé tous les deux jours.

On enlève le milieu de culture complet et on rince avec du milieu seul. On prive les cellules pendant 17 heures (milieu seul + 10 % de SVF, thymidine tritiée, 10 µCi/ml (Réf : TRK 758 AMERSHAM), soit 10 µL de ³HT/Volume de milieu complet). On élimine le surnageant (à conserver pour élimination ultérieure), puis on enlève le matériau de l'agarose et on le transfère dans un puits en plastique. On rince deux fois avec du PBS 1X (ou du Hank's 1X), puis on fixe les cellules avec du méthanol froid 100 %, pendant 10 min à 4°C. On rince deux fois avec du PBS 1X (ou du Hank's 1X), et on précipite avec du TCA froid 5 % pendant 20 minute à 4°C. On élimine le surnageant, et on rince quatre fois avec du PBS 1X (ou du Hank's 1X).

On incube dans NaOH 0,3 N pendant 2 heures à température ambiante (dissolution de la couche cellulaire), sans dépasser 300 à 500 µL de NaOH. On récupère la solution dans un tube à scintillation auquel on rajoute 5 ml de liquide scintillant, et on détermine la radioactivité de la solution et du matériau à l'aide d'un compteur β (radioactivité négligeable).

A titre de comparaison, la même expérience est réalisée sur un échantillon de corail brut (aragonite) et sur un échantillon de produit BIOCORAL®.

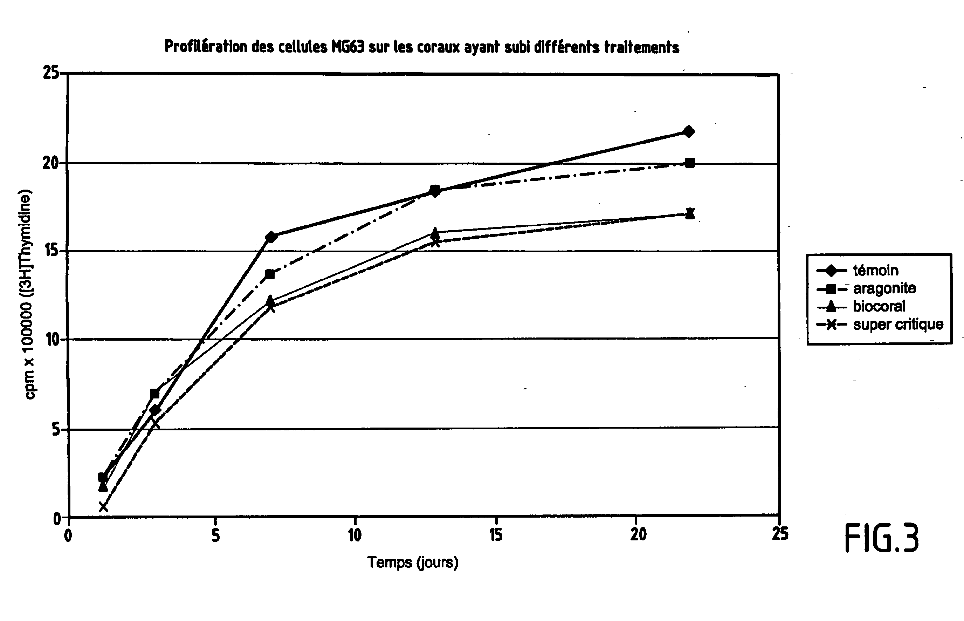

Les résultats sont présentés sur la Figure 3. On peut constater que la prolifération cellulaire des cellules MG63 sur le corail traité selon l'invention, est identique à celle observée sur le produit BIOCORAL®.

L'ensemble de ces résultats suggère que le corail traité selon la présente invention peut être utilisé dans la fabrication de substituts osseux.

## Revendications

1. Procédé d'extraction de substances organiques présentes dans le corail, qui consiste à traiter le corail par un fluide ou un mélange de fluides à l'état supercritique sans modifier la structure cristalline dudit corail, à une température inférieure à 270°C, de préférence inférieure ou égale à 260°C, et de préférence encore inférieure ou égale à 250°C et à une pression très supérieure à la pression critique dudit fluide ou mélange de fluides, par exemple de l'ordre d'au moins 3 fois, et de préférence d'au moins 5 fois ladite pression critique.

2. Procédé selon la revendication 1, dans lequel ledit fluide est choisi parmi l'éthanol et l'acétone, et le mélange de fluides est choisi parmi les mélanges éthanol-dioxyde de carbone et acétone-dioxyde de carbone dont la température critique est inférieure à 270°C, de préférence inférieure ou égale à 260°C, et de préférence encore inférieure ou égale à 250°C.

3. Procédé selon la revendication 1 ou 2, dans lequel ledit fluide est l'éthanol.

4. Procédé selon la revendication 3, dans lequel la pression de traitement du corail est comprise entre 300 et 450 MPa, de préférence entre 350 et 400 MPa.

5. Procédé selon la revendication 4, dans lequel la température de traitement du corail est comprise entre 240 et 260°C, de préférence de l'ordre de 250°C et de le temps de traitement du corail est compris entre 15 et 240 min, de préférence de l'ordre de 1 h.

6. Procédé selon la revendication 2, dans lequel le mélange de fluides est un mélange éthanol-dioxyde de carbone, la pression de traitement du corail est comprise entre 30 et 50 MPa, de préférence de l'ordre de 40 MPa.

7. Procédé selon la revendication 6, dans lequel la température de traitement du corail est de l'ordre de 80 à 100°C.

8. Corail obtenu par le procédé selon l'une des revendications 1 à 7.

9. Substitut osseux fabriqué à partir du corail selon la revendication 8.

## Claims

1. A method of extracting organic substances present in coral, which consists in treating the coral with a fluid or a mixture of fluids in the supercritical state without modifying the crystalline structure of said coral, at a temperature of less than 270°C, preferably 260°C or less, and more preferably 250°C or less, and at a pressure which is much higher than the critical pressure of said fluid or mixture of fluids, for example of the order of at least 3 times, and preferably at least 5 times said critical pressure.

2. A method according to claim 1, in which said fluid is selected from ethanol and acetone,and the mixture of fluids is selected from an ethanol and carbon dioxide mixture and from an acetone and carbon dioxide mixture, the critical temperature of which is less than 270°C, preferably 260°C or less, and more preferably 250°C or less.

3. A method according to claim 1 or claim 2, in which said fluid is ethanol.

4. A method according to claim 3, in which the coral treatment pressure is in the range 300 MPa to 450 MPa, preferably in the range 350 MPa to 400 MPa.

5. A method according to claim 4, in which the coral treatment temperature is in the range 240°C to 260°C, preferably of the order of 250°C and the coral treatment period is in the range 15 min to 240 min, preferably of the order of 1 hour.

6. A method according to claim 2, in which the mixture of fluids is an ethanol and carbon dioxide mixture, and the coral treatment pressure is in the range 30 MPa to 50 MPa, preferably of the order of 40 MPa.

7. A method according to claim 6, in which the coral treatment temperature is of the order of 80°C to 100°C.

8. Coral obtained by the method according to any one of claims 1 to 7.

9. A bone substitute fabricated from coral in accordance with claim 8.

## Patentansprüche

1. Verfahren zur Extraktion von organischen Substanzen, die in der Koralle vorliegen, welches darin besteht, die Koralle durch ein Fluid oder ein Fluidgemisch im superkritischen Zustand zu behandeln, ohne die Kristallstruktur der Koralle zu modifizieren, bei einer Temperatur unter 270°C, vorzugsweise unter oder gleich 260°C und weiter bevorzugt unter oder gleich 250°C und bei einem Druck, der weit über dem kritischen Druck des Fluids oder Fluidgemischs liegt, zum Beispiel in der Größenordnung von wenigstens 3 mal und vorzugsweise wenigstens 5 mal dem kritischen Druck.

2. Verfahren nach Anspruch 1, bei dem das Fluid gewählt wird aus Ethanol und Aceton und das Fluidgemisch gewählt wird aus den Gemischen Ethanol-Kohlendioxyd und Aceton-Kohlendioxyd, deren kritische Temperatur unter 270°C, vorzugsweise unter oder gleich 260°C und weiter bevorzugt unter oder gleich 250°C liegt.

3. Verfahren nach Anspruch 1 oder 2, bei dem das Fluid Ethanol ist.

4. Verfahren nach Anspruch 3, bei dem der Druck zur Behandlung der Koralle zwischen 300 und 450 MPa liegt, vorzugsweise zwischen 350 und 400 MPa.

5. Verfahren nach Anspruch 4, bei dem die Temperatur zur Behandlung der Koralle zwischen 240 und 260°C, vorzugsweise in der Größenordnung von 250°C liegt und die Zeit zur Behandlung der Koralle zwischen 15 und 240 Minuten, vorzugsweise in der Größenordnung von einer Stunde liegt.

6. Verfahren nach Anspruch 2, bei dem das Fluidgemisch ein Ethanol-Kohlendioxyd- Gemisch ist und der Druck zur Behandlung der Koralle zwischen 30 und 50 MPa, vorzugsweise in der Größenordnung von 40 MPa liegt.

7. Verfahren nach Anspruch 6, bei dem die Temperatur zur Behandlung der Koralle in der Größenordnung von 80 bis 100°C liegt.

8. Koralle, erhalten durch das Verfahren nach einem der Ansprüche 1 bis 7.

9. Knochenersatz, hergestellt aus der Koralle nach Anspruch 8.
